Europäisches Patentamt

European Patent Office

Office européen des brevets

(19)

(11) Numéro de publication: **0 140 438 B1**

(12) **FASCICULE DE BREVET EUROPEEN**

(45) Date de publication du fascicule du brevet:
06.09.89

(51) Int. Cl.⁴: **C 07 D 213/61**

(21) Numéro de dépôt: **84201470.6**

(22) Date de dépôt: **12.10.84**

(54) Procédé pour la préparation de la 2-chloropyridine.

(30) Priorité: **24.10.83 FR 8317032**

(43) Date de publication de la demande:
**08.05.85 Bulletin 85/19**

(45) Mention de la délivrance du brevet:
**06.09.89 Bulletin 89/36**

(84) Etats contractants désignés:
**AT BE CH DE FR GB IT LI LU NL SE**

(56) Documents cités:
**EP-A- 0 013 474**
**US-A- 3 153 044**
**US-A- 3 173 919**
**US-A- 4 054 499**

**J.K. KOCHI, Free Radicals, John Wiley & Sons, Vol.II, 1973, p.174**
**KIRK-OTHMER, ECT, Vol. 13, John Wiley & Sons, 1981, p.355**
**J. Heterocyclic Chem. 1967, 375-76**

**Le dossier contient des informations techniques présentées postérieurement au dépôt de la demande et ne figurant pas dans le présent fascicule.**

(73) Titulaire: **SOLVAY & Cie (Société Anonyme), Rue du Prince Albert, 33, B-1050 Bruxelles (BE)**

(72) Inventeur: **Walraevens, René, Avenue des Neuf Provinces, 3, B-1080 Bruxelles (BE)**
Inventeur: **Franklin, James, Rue Edouard Olivier, 28, B-1170 Bruxelles (BE)**
Inventeur: **Trouillet, Paul, Avenue du Château, 8, B-1080 Bruxelles (BE)**

ACTORUM AG

## Description

La présente invention concerne un procédé pour la préparation de la 2-chloropyridine par chloration de la pyridine en phase vapeur en présence de catalyseurs.

A ce jour, différentes techniques ont été développées pour la préparation des 2-halopyridines telles que la 2-chloropyridine. Une de ces techniques consiste plus particulièrement dans la préparation de la 2-chloropyridine par une réaction de chloration de la pyridine au moyen de chlore moléculaire. Cette technique a fait l'objet de mises au point de procédés pouvant être répartis en deux grandes classes.

Une première classe comprend les procédés dits thermiques, effectués en phase vapeur à haute température, généralement supérieure à 250°C, voire même 300°C à 400°C et décrits notamment dans les brevets Etats-Unis 2 820 791 et 3 153 044 au nom de OLIN MATHIESON, et dans le brevet Etats-Unis 3 920 657 au nom de Deutsche Gold- und Silber-Scheideanstalt vormals Roessler. Ces procédés présentent divers désavantages tels qu'une formation importante de goudrons, ce qui engendre des bouchages du réacteur ou des conduites, et rend dès lors difficile l'exécution du procédé en continu. En outre, ces procédés s'accompagnent d'après les inventeurs de risques d'explosion ainsi que de corrosion élevées.

Une deuxième classe de procédés comprend les procédés initiés au moyen de rayonnements lumineux ou ultraviolets. De tels procédés ont été notamment décrits dans les brevets Etats-Unis 3 297 556 au nom de OLIN MATHIESON et 4 054 499 au nom de SEITETSU KAGAKU Co et dans le document J. Het. Chem. 4, 375 (1967).

Ces procédés, bien que permettant d'opérer à des températures plus basses que les procédés dits thermiques présentent l'inconvénient de conduire à la formation de sous-produits goudronneux qui salissent les tubes lumineux et donnent lieu à une diminution subséquente du rayonnement d'initiation et dès lors du rendement de la réaction. En outre ces procédés nécessitent d'opérer en réacteurs perméables aus rayonnements d'initiation, c'est-à-dire le plus généralement en verre, ce qui rend inexploitable des réactions réalisées à pressions élevées.

Par ailleurs un cas particulier de cette deuxième classe est constitué par le procédé décrit dans la demande de brevet européen EP-A-0 013 474. En effet cette demande de brevet décrit un procédé de fabrication de dérivés pyridiniques particuliers tels que la 2-chloro-5-trifluorométhyl-pyridine ou la 2-chloro-5-perchlorofluorométhyl-pyridine par chloration de respectivement la 3-trifluorométhylpyridine ou la 3-perchlorofluorométhylpyridine dans lequel la chloration peut être soit effectuée en phase vapeur, soit en phase liquide. Lorsque le procédé est effectué en phase vapeur la chloration de ces produits est initiée soit thermiquement soit par des rayonnements UV. Par contre lorsque le procédé est effectué en phase liquide les initiateurs sont soit des rayonnements UV soit des initiateurs thermiques libérant des radicaux libres.

La présente invention a pour but de fournir une nouvelle classe de procédés permettant de fabriquer la 2-chloropyridine et en général les 2-halopyridines sans encourir les désavantages des procédés des deux classes décrites ci-dessus.

L'invention concerne un procédé pour la préparation de la 2-chloropyridine par une réaction de chloration de la pyridine au moyen de chlore moléculaire, effectuée en phase vapeur à des températures comprises entre 175 et 275°C, en présence d'additifs tels que la vapeur d'eau et d'additifs organiques tels que le tétrachlorure de carbone, et à l'intervention de catalyseurs, dans laquelle on met en œuvre comme catalyseurs au moins un composé organique capable de générer des radicaux libres dans les conditions de la réaction de chloration.

Par réaction de chloration effectuée en phase vapeur, la Demanderesse entend définir une réaction de chloration réalisée dans des conditions de température et de pression telles que tous les réactifs, additifs, catalyseurs et produits issus de la réaction se trouvent à l'état de vapeur dans le milieu réactionnel.

Lorsque l'on opère à pression atmosphérique, ces conditions sont remplies pour des températures supérieures à 150°C. De bons résultats ont été obtenus à des températures situées entre 175 et 275°C et plus particulièrement entre 200 et 250°C.

Par composés organiques capables de générer des radicaux libres dans les conditions de la réaction de chloration, on entend tout composé capable de se décomposer avec formation d'au moins un radical organique aux température et pression telles que la réaction de chloration ait lieu en phase vapeur. De tels composés organiques comprennent non seulement les produits chimiques constitués uniquement d'entités organiques tels que les peroxydes organiques ou des dérivés azotés tels que les azoalkanes ou nitriles du type azo-bis-isobutyronitrile mais également des produits chimiques comprenant une partie inorganique dans la molécule tels que les peroxydes mixtes organiques-inorganiques et les dérivés organo-métalliques tels que le plomb tétraéthyle.

Habituellement la réaction de chloration est catalysée par des peroxydes ou dérivés peroxydés de type organique. Parmi ceux-ci conviennent bien les hydroperoxydes, les alpha-oxy- et alpha-peroxyhydroperoxydes et -peroxydes, les peroxydes, les diacylperoxydes, les peroxyacides et les peroxyesters tels que notamment ceux définis dans l'ouvrage Encyclopedia of Chemical Technology Kirk-Othmer Third Edition Volume 17 pages 27 à 89 édité par J. Wiley & Sons New-York 1982. Parmi ces produits peroxydés, de bons résultats ont été obtenus avec des peresters d'alkyle d'acides aliphatiques ou aromatiques et avec des peroxydes de dialkyle. Les meilleurs résultats ont été obtenus avec du perbenzoate de tertiaire butyle, le per-2-éthylhexanoate de tertiaire butyle, le perpivalate de tertiaire butyle, le perpivalate de tertiaire amyle et le peroxyde de di-tertiaire butyle. Enfin parmi ceux-ci c'est le peroxyde de di-tertiaire butyle qui a donné les meilleurs résultats.

La quantité de composés organiques capables de générer des radicaux libres mise en œuvre dépend de la nature chimique du composé organique utilisé. Lorsque l'on travaille avec des peroxydes tels que le peroxyde de di-tertiaire butyle on met généralement en œuvre de 0,05 à 5 mol% de ce peroxyde calculé par rapport à la pyridine mise en œuvre; les quantités préférées se situent entre 0,1 et 2 mol% par rapport à la pyridine mise en œuvre.

Le chlore moléculaire et la pyridine sont habituellement mis en œuvre dans des rapports molaires compris entre 0,1 à 10 moles de chlore par mole de pyridine. De préférence on met en œuvre de 0,2 à 2 moles de chlore par mole de pyridine; afin tout particulièrement préférés sont des rapports molaires chlore/pyridine situés entre 0,3 et 1 mole de chlore par mole de pyridine.

Outre les réactifs et catalyseurs déjà cités on peut avantageusement mettre en œuvre dans le procédé selon l'invention des additifs tels que de la vapeur d'eau, de l'azote et/ou d'autres gaz ne participant pas à la réaction de chloration proprement dite. Habituellement on réalise la réaction de chloration en présence de vapeur d'eau à raison de 0,1 à 25 moles par mole de pyridine mise en œuvre. De préférence on utilise des quantités de vapeur d'eau de 1 à 15 moles par mole de pyridine. L'adjonction de ces additifs et plus particulièrement de la vapeur d'eau peut être réalisée de toutes les façons. Une façon avantageuse consiste à mélanger préalablement l'eau et la pyridine, à envoyer ce mélange dans un évaporateur et à injecter ensuite les vapeurs ainsi obtenues dans le réacteur de chloration proprement dit.

On a également constaté qu'il est souhaitable pour assurer une bonne homogénéisation des réactifs et des catalyseurs d'exécuter la réaction de chloration en présence d'additifs de nature organique qui agissent comme solvants ou diluants mais qui sont inertes vis-à-vis des réactifs et des catalyseurs intervenant dans la réaction de chloration. Comme additifs organiques on utilise habituellement des composés organiques halogénés. Parmi ceux-ci, on opère de préférence avec des dérivés chlorés de composés aliphatiques pouvant être utilisés conjointement à la vapeur d'eau dans le milieu réactionnel afin de minimiser la surchauffe des réactifs et du réacteur du fait de l'exothermicité de la réaction de chloration.

Comme dérivés chlorés convenant à titre d'additifs organiques, on peut utiliser des produits tels que le tétrachlorure de carbone ou d'autres dérivés halogénés inertes dans les conditions de chloration. De préférence on opère avec le tétrachlorure de carbone.

En général les additifs organiques halogénés sont ajoutés au milieu réactionnel à raison de 1 à 25 moles par mole de pyridine mise en œuvre. Lorsque l'on utilise du tétrachlorure de carbone, comme additif organique, les quantités préférées se situent entre 1,5 et 10 moles par mole de pyridine mise en œuvre.

Le procédé selon l'invention peut être réalisé dans tout appareillage ou tout réacteur permettant de réunir les conditions opératoires décrites ci-avant. De bons résultats ont été obtenus dans des appareillages permettant l'obtention d'une bonne homogénéisation des réactifs avec les catalyseurs et les divers additifs, ainsi qu'une introduction des réactifs et des catalyseurs à des vitesses relativement élevées.

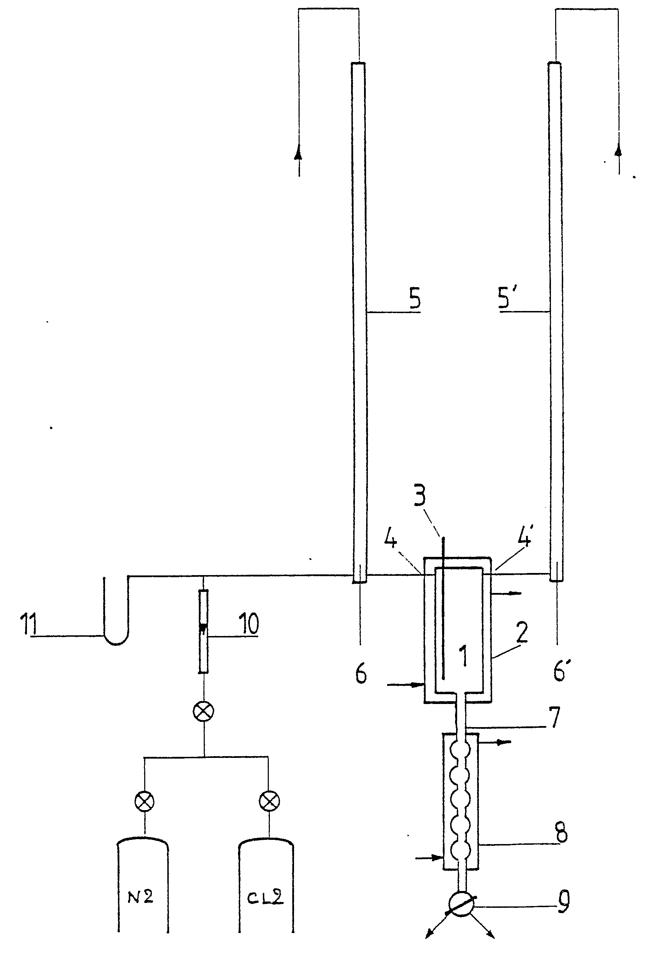

Un appareillage de laboratoire satisfaisant à ces critères est représenté à la figure annexée. Cet appareillage comprend un tube cylindrique, d'un diamètre de 112 mm et d'une hauteur de 280 mm, formant une enceinte réactionnelle (1) et une enveloppe (2) qui est chauffée par circulation d'huile au travers de l'enveloppe (2). L'enceinte réactionnelle (1) est équipée d'une gaine pour thermocouples (3), de diamètre externe de 8 mm, permettant de mesurer la température en différents points. Le haut de l'enceinte réactionnelle est équipé de deux entrées horizontales de 6 mm de diamètre (4) et (4') placées à 90° l'une de l'autre et permettant d'injecter tangentiellement les réactifs vaporisés. Les produits gazeux sont admis à sortir de l'enceinte réactionnelle par le bas au travers du tube (7) d'un diamètre de 20 mm.

Les réactifs, additifs et catalyseurs liquides comprenant d'une part une solution de peroxyde organique dans le tétrachlorure de carbone et d'autre part la pyridine et l'eau sont alimentés respectivement en tête de deux tubes en acier inoxydable verticaux (5) et (5') d'une hauteur de 1,5 m et d'un diamètre intérieur de 15 mm dans lesquels ils sont vaporisés par chauffage électrique. La température des vapeurs est ensuite mesurée respectivement au moyen des thermocouples (6) et (6') montés dans les pieds des tubes en acier inoxydable, près des entrées (4) et (4') de l'enceinte réactionnelle, auxquelles les tubes en acier inoxydable (5) et (5') sont reliés au moyen de raccords en TEFLON.

Le chlore gazeux moléculaire, éventuellement additionné d'azote, dont le débit est mesuré au moyen d'un rotamètre (10) et d'un manomètre à mercure (11), est injecté tel quel dans le pied du tube (5), dans lequel le tétrachlorure de carbone est vaporisé.

Les produits réactionnels gazeux issus de la réaction sont éliminés au travers du tube (7) et sont ensuite condensés dans un condenseur en pyrex (8) de 300 mm de long équipé d'une jacquette de refroidissement à eau et d'un robinet en pyrex à 2 voies (9).

Les produits condensés recueillis par le robinet (9) peuvent subir différents traitements et peuvent notamment être purifiés. Une façon d'opérer consiste à agiter violemment les produits de condensation avec une quantité suffisante d'un réactif basique tel que le NaOH pour neutraliser tout le chlore moléculaire et l'acide chlorhydrique résiduaire. Après décantation du liquide il se forme deux phases constituées d'une part, d'une phase lourde organique, comprenant le tétrachlorure de carbone, une partie de la pyridine résiduaire ainsi que les produits de chloration tels que la 2-chloropyridine et une phase légère aqueuse contenant le restant de la pyridine n'ayant pas réagi ainsi que la plupart des produits ou ions de nature non organique. Les phases sont séparées et la pyridine de la phase aqueuse est récupérée par extraction au moyen d'un solvant généralement halogéné tel que le tétrachlorure de carbone et de préférence le chloroforme. Les différentes phases organiques sont ensuite réunies et la 2-chloropyridine peut être séparée de ce milieu par une opération de rectification.

La 2-chloropyridine obtenue selon le procédé de l'invention peut être utilisée dans toutes les applications connues de ce produit c'est-à-dire comme intermédiaire chimique pour la fabrication notamment de produits destinés à l'agriculture, de cosmétiques et de produits pharmaceutiques.

La réaction de chloration de la pyridine peut bien entendu, moyennant des mises au point techniques évidentes propres aux différents produits concernés, être appliquée à d'autres composés hétérocycliques aromatiques.

Les exemples qui suivent servent à illustrer l'invention.

Exemple 1

Dans un appareil tel qu'illustré à la figure et ayant les caractéristiques décrites ci-avant, on introduit en continu par le tube (5), maintenu à 144 °C, 8,2 moles de tétrachlorure de carbone mélangés à 0,041 mole de peroxyde de di-tertiaire butyle par heure et par le tube (5'), maintenu à 140 °C, 3,27 moles de pyridine et 3,50 moles d'eau par heure. En outre on injecte en continu dans le pied du tube (5), 2,18 moles de chlore moléculaire gazeux par heure. Les réactifs, catalyseur et additifs évaporés sont alors introduits en continu par les entrées (4) respectivement (4') dans l'enceinte réactionnelle (1) chauffée à environ 240 °C au moyen d'huile circulant dans l'enveloppe (2). Après 1 heure de mise en régime on mesure 242 °C en haut, 244 °C au milieu et 241° C dans le bas de l'enceinte réactionnelle et on recueille ensuite en continu et par heure par la voie 9 un mélange de liquide ayant la composition suivante:

| 2-chloropyridine: | 1,136 mole |
|---|---|
| 3-chloropyridine: | 0,006 mole |
| 4-chloropyridine: | 0,004 mole |
| 2,6-dichloropyridine: | 0,034 mole |
| autres dichloropyridines: | 0,002 mole |
| pyridine: | 2,089 moles |

Le temps de séjour des réactifs dans le réacteur est de 13 sec et le rendement de la réaction en 2-chloropyridine est de 35% par mole calculé par rapport à la pyridine mise en œuvre; la sélectivité des produits chlorés issus de la chloration est de 96% en ce qui concerne la 2-chloropyridine.

Exemples 2, 3 et 4

On opère comme décrit à l'exemple 1 mais avec des quantités de réactifs, additifs et catalyseur et des températures tels que repris au tableau ci-après. Les résultats observés sont également repris au tableau.

| Paramètres | | Exemple 2 | Exemple 3 | Exemple 4 |
|---|---|---|---|---|
| Alimentation en | mole/heure | | | |
| (5) du tétrachlorure de carbone | | 4,05 | 4,04 | 4,05 |
| (5) du peroxyde de ditertiaire butyle | | 0,0204 | 0,0102 | 0,005 |
| (5') de la pyridine | | 1,59 | 1,59 | 1,60 |
| (5') de l'eau | | 1,71 | 1,74 | 1,66 |
| pied du tube (5) de chlore | | 1,09 | 1,09 | 1,09 |
| | | | | |
| Températures | °C | | | |
| du tube (5) | | 151 | 151 | 135 |
| du tube (5') | | 166 | 151 | 144 |
| de l'enceinte réactionnelle (1) | | | | |
| – dans le haut | | 232 | 232 | 231 |
| – au milieu | | 232 | 233 | 234 |
| – dans le bas | | 231 | 231 | 233 |
| Temps de séjour des réactifs en sec. | | 26 | 26 | 26 |
| | | | | |
| Produits recueillis par la voie (9) | mole/heure | | | |
| 2-chloropyridine | | 0,594 | 0,607 | 0,574 |
| 3-chloropyridine | | 0,003 | 0,003 | 0,003 |
| 4-chloropyridine | | 0,002 | 0,002 | 0,002 |
| 2,6-dichloropyridine | | 0,017 | 0,019 | 0,016 |
| autres dichloropyridines | | 0,001 | 0,001 | 0,001 |
| pyridine | | 0,974 | 0,959 | 1,005 |

Les rendements obtenus en 2-chloropyridine sont respectivement de 37% (exemple 2), 38% (exemple 3) et 36% (exemple 4) par mole calculés par rapport à la pyridine. Les sélectivités respectives sont de 96% (exemple 2), 96% (exemple 3) et 96% (exemple 4).

La comparaison des résultats des exemples 1 à 4 permet de constater que la réaction de chloration de la pyridine en 2-chloropyridine est déjà catalysée par de faibles quantités de peroxydes et n'est que très peu affectée par des variations de 100 à 200% de cette teneur.

## Revendications

1. Procédé de préparation de la 2-chloropyridine par une réaction de chloration de la pyridine au moyen de chlore moléculaire effectuée en phase vapeur à des températures comprises entre 175 et 275 °C, en présence d'additifs tels que la vapeur d'eau et d'additifs organiques tels que le tétrachlorure de carbone et à l'intervention de catalyseurs caractérisé en ce que l'on met en œuvre comme catalyseurs au moins un composé organique capable de générer des radicaux libres dans les conditions de la réaction de chloration.

2. Procédé selon la revendication 1, caractérisé en ce que l'on met en œuvre comme composé organique capable de générer des radicaux libres au moins un composé appartenant à la classe des peroxydes.

3. Procédé selon la revendication 2, caractérisé en ce que le composé appartenant à la classe des peroxydes est choisi parmi les peresters d'alkyle d'acides aliphatiques ou aromatiques ou encore les peroxydes de dialkyle.

4. Procédé selon la revendication 3, caractérisé en ce que le peroxyde de dialkyle est le peroxyde de di-tertiaire butyle.

5. Procédé selon la revendication 4, caractérisé en ce qu'on met en œuvre le peroxyde de di-tertiaire butyle à raison de 0,05 à 5 mol% calculés par rapport à la pyridine.

## Claims

1. Process for the preparation of 2-chloropyridine by a chlorination reaction of pyridine by means of molecular chlorine, carried out in the vapour phase at temperatures of between 175 and 275 °C, in the presence of additives such as water vapour and of organic additives such as carbon tetrachloride, and employing catalysts, characterized in that as catalysts there is used at least one organic compound capable of generating free radicals under the chlorination reaction conditions.

2. Process according to Claim 1, characterized in that as the organic compound capable of generating free radicals there is used at least one compound belonging to the class of the peroxides.

3. Process according to Claim 2, characterized in that the compound belonging to the class of the peroxides is chosen from among the alkyl peresters of aliphatic or aromatic acids or among the dialkyl peroxides.

4. Process according to Claim 3, characterized in that the dialkyl peroxide is di-tertiary butyl peroxide.

5. Process according to Claim 4, characterized in that di-tertiary butyl peroxide is employed in an amount of 0.05 to 5 mol% calculated relative to the pyridine.

## Patentansprüche

1. Verfahren zur Herstellung von 2-Chloropyridin durch eine Chlorierungsreaktion des Pyridins mit molekularem Chlor, durchgeführt in der Dampfphase bei einer Temperatur zwischen 175 und 275 °C in Anwesenheit von Zusatzstoffen wie Wasserdampf und organischen Zusatzstoffen wie Kohlenstofftetrachlorid und unter Einsatz von Katalysatoren, dadurch gekennzeichnet, daß man als Katalysator wenigstens eine organische Verbindung einsetzt, die unter den Bedingungen der Chlorierungsreaktion freie Radikale erzeugen kann.

2. Verfahren nach Anspruch 1, dadurch gekennzeichnet, daß man als organische Verbindung, die freie Radikale erzeugen kann, wenigstens eine Verbindung einsetzt, die zur Klasse der Peroxyde gehört.

3. Verfahren nach Anspruch 2, dadurch gekennzeichnet, daß die zur Klasse der Peroxyde gehörende Verbindung ausgewählt ist unter den Alkylperestern aliphatischer oder aromatischer Säuren oder unter den Dialkylperoxyden.

4. Verfahren nach Anspruch 3, dadurch gekennzeichnet, daß das Dialkylperoxyd Di-tertiär-butyl-peroxyd ist.

5. Verfahren nach Anspruch 4, dadurch gekennzeichnet, daß man das Di-tertiär-butyl-peroxyd in einer Menge von 0,05 bis 5 Mol-%, bezogen auf das Pyridin, einsetzt.